# EUROPEAN PATENT APPLICATION

(11) **EP 1 842 565 A1**
(43) Date of publication of application: **10.10.2007**
(21) Application number: 06112262.8
(22) Date of filing: 05.04.2006
(51) Int. Cl.: A61L 15/46

(54) **Absorbent articles including odour control system**

(71) Applicant: THE PROCTER & GAMBLE COMPANY, Cincinnati, Ohio 45202 (US)
(72) Inventor: Carlucci, Giovanni, 66100 Chieti (IT); Pesce, Antonella, 65125 Pescara (IT); Caputi, Mariangela, 70056 Molfetta (BA) (IT); Sierri, Giancarlo, 64030 Montefino (TE) (IT)
(74) Representative: Kremer, Véronique Marie Joséphine

(57) **Abstract**

Absorbent articles which are provided with an odour control system. The odour control system includes at least two classes of odour control materials, wherein one class acts on malodours or a malodorous substance in the absorbent article, whereas a second class acts on nose receptors. The classes of odour control materials may be selected to provide a synergistic effect in terms of malodour reduction.

## Description

### FIELD OF THE INVENTION

The present invention relates to absorbent articles which are provided with an odour control system. The odour control system comprises classes of odour control materials, wherein one class reduces malodours by acting on the malodours or malodorous substance and a second class acts on nose receptors. The classes of odour control materials may be selected to provide a synergistic effect in terms of malodour reduction.

### BACKGROUND OF THE INVENTION

Absorbent articles of personal hygiene are known in the art. Typical examples include sanitary napkins, panty liners, adult incontinence articles, infant diapers, paper towels, bath tissue and facial tissue. Such articles are often used to absorb and retain bodily fluids and other exudates excreted by the human body. Typically, such exudates are perceived as malodorous and offensive. Therefore, methods and materials for controlling and reducing malodours in absorbent articles have been developed. Some examples are discussed hereinafter.

An early, basic reference in this respect is EP 510 619. This document discloses a wide variety of materials, which have proven to be effective in certain circumstances in reducing malodours in absorbent articles of personal hygiene. EP 959 846 discloses such materials comprising polyacrylate superabsorbers and silica. EP 811 387 discloses absorbent articles being provided with a zeolite and silica odour control system. EP 963 186 discloses an odour control systems comprising zeolites, silica and polyacrylic superabsorbers. EP 912 149 discloses chelating agents for use in odour control in absorbent articles, particularly polyfunctionally substituted aromatic chelants.

All of the above solutions can provide a consumer-noticeable degree of malodour reduction in absorbent articles. However, due to the nature of action and the materials chosen only a limited variety of malodorous compounds can be counteracted.

Therefore it is desirable to provide absorbent articles having an odour control system, which acts against a wide variety of malodours in a holistic manner. It is also desirable to provide an absorbent article having an odour control system including at least one class of materials that reduces malodours by acting on malodours or a malodourous substance in the article and/or at least one class that acts on certain nose receptors. It would also be desirable to provide an absorbent article including an odour control system having at least one material that acts on the malodours and/or malodorous substance to reduce the odour and the same or another material that acts on nose receptors to help reduce the perception of malodour.

### SUMMARY OF THE INVENTION

The present invention addresses the above need by providing an odour control system, which provides an absorbent article comprising an odour control system, wherein the odour control composition comprises at least two classes of odour control materials. The first class of odour control material reduces odour by acting on malodours or a malodorous substance in the absorbent article and a second class of odour control material reduces odour by acting on the user's nose receptors. The absorbent article is characterized in that the first class of odour control material is selected from the group consisting of silica gel having a pH of less than 7, aldehydes, mesoporous zeolites having pores sizes of from 20 to 500 Å and mixtures thereof, wherein said aldehydes are selected from the group consisting of α-amylcinnamic aldehyde, p-anisaldheyde, benzaldehyde, cinnamic aldehyde, cuminic aldehyde, decanal, p-t-Butyl-alpha-methyldihydrocinnamaldehyde, 4-Hydroxy-3-methoxycinnamaldehyde, 2-Phenyl-3-(2-furyl)prop-2-enal, Vanillin isobutyrate, 2-Phenyl-3-(2-furyl)prop-2-enal, Ethyl vanillin acetate, Vanillin acetate, cyclamen aldehyde, heptanal, lauryl aldehyde, nonanal, octanal, phenylacetaldehyde, phenyl propyl aldehyde, vanillin, salycil aldehyde, cytral and mixtures thereof, and the second class of odour control material is selected from the group consisting of menthol, menthyl acetate, 3-Buten-2-one,3-methyl-4-(2,6,6-trimethyl-2-cyclohexen-1-yl)-, 4-(2,6,6-Trimethylcyclohen-1-en-1-yl)but-3-en-2-one, 3-Buten-2-one,4-(2,6,6-trimethyl-2-cyclohexen-1-yl)-,(E)-, menthyl lactate, isomenthyl acetate, isomenthyl propionate, isomenthyl isobutyrate, isomenthyl propionate, isomenthyl butyrate, camphor, p-menthane and mixtures thereof.

### DETAILED DESCRIPTION OF THE INVENTION

The term "absorbent article" is used herein in a very broad sense including any article able to receive and/or absorb and/or contain and/or retain fluids and/or exudates, especially bodily fluids/bodily exudates. Exemplary absorbent articles in the context of the present invention are disposable absorbent articles. The term "disposable" is used herein to describe articles, which are not intended to be laundered or otherwise restored or reused as an article (i.e. they are intended to be discarded after a single use and preferably to be recycled, composted or otherwise disposed of in an environmentally compatible manner). Typical disposable absorbent articles according to the present invention are diapers, surgical and wound dressings and perspiration pads, incontinence pads, as well as absorbent articles for feminine hygiene like sanitary napkins, panty liners, tampons, interlabial devices or the like. Certain absorbent articles include a fluid pervious topsheet, a fluid impervious backsheet that is preferably water vapour and/or gas pervious and an absorbent core comprised there between.

The term "use", as used herein, refers to the period of time that starts when the absorbent article is actually put in contact with the anatomy of a wearer. It is further underlined that the odour neutralization benefits provided by the absorbent articles of the present invention are already provided as soon as the absorbent articles is released from its package. This is due to the volatility of the odour neutralization materials acting on nose receptors (sometimes referred to herein as acting "externally"), which are further specified below.

By "body fluid" it is meant herein any fluid produced by human body including, but not limited to, perspiration, urine, menstrual fluids, vaginal secretions and the like.

The present invention relates to an odour control system, which reduces the malodour generated by a broad range of odorous materials typically occurring in or resulting from the degradation of body fluids and/or materials making up the absorbent article. The odour control system herein includes at least two classes of odour control materials; one class of odour control materials counteracts the malodours of the exudates or other malodorous material (also referred to herein as acting "internally"), whereas a second class counteracts such malodours by affecting the receptors in the nose.

### Odour control materials acting on the malodours or malodorous substance

There are many materials known in the art for counteracting malodours in absorbent articles. Examples can be found in the references cited herein before. Typical substances are zeolites, starch, activated carbon, cyclodextrine, chitin or chitosan and esters.

These actives can reduce the malodour unpleasantness according to different mechanisms, e.g. they can reduce the amount of malodorous molecules through absorption/adsorption mechanisms and/or can react with the malodorous molecules transforming them into low volatile/non-odorous ones and/or can suppress malodorous molecules volatility and/or can prevent the malodour generation by inhibiting degradative processes caused by microorganisms metabolic activity.

For the odour control system of the present invention a specific selection of such materials is desired. It has been found that silica gel, aldehydes and mesoporous zeolites are particularly useful.

Silica gel is a porous, amorphous form of silica (SiO₂). It is composed of a vast network of interconnected microscopic pores. As opposed to zeolites, silica gels have larger pores with a wide range of diameters typically between 5 Å and 3000 Å.

Silica gel, which has proven particularly useful in the odour control system herein is the narrow silica gel with a pH of less than 7. Indeed it was discovered that this type of silica gel is effective in reducing the malodour level according to two different types of odour control mechanisms: absorption/adsorption of malodorous molecules on silica surface and neutralization of aminic components. The latter are a main source of malodour especially in feminine hygienic products. Silica gels with a pH of less than 6 may be desirable for certain embodiments.

It is possible to adjust the silica gel pore size range in the manufacturing process: Silica gels synthesized with an average pore size of about 10-50Å are known as "narrow" pore silica gels; silica gels with an average pore size of about 110 Å and beyond are called "wide" pore silica gels. Silica gels with wide pore are generally more expensive than narrow silica gel. In certain embodiments, the silica gel used herein is the narrow silica gel with average pore size of from 20 - 40 Å, or in some embodiments 30Å.

One suitable silica gel herein has a total surface area higher than 500 m²/g. The total surface area can be established by using the BET (Brunauer-Emmett-Teller) test. This test is based on the adsorption of nitrogen gas at 77 K onto the surfaces of the silica gel particles, i.e. also their internal cavities. The adsorbed volume of nitrogen is then established by comparing the nitrogen pressure before and after the adsorption. Exemplary suitable silica gel materials include silica gel code 122 and 123 available from Grace, Columbia, MD, USA.

It has further been found by the present inventors that narrow silica gel or zeolites, especially mesoporous zeolites, can be used to stabilize the volatile odour control materials acting externally in the absorbent article. Mesoporous zeolites are those zeolites with pore size from 20 to 500 Å. As indicated above, silica gel can absorb volatile substances and thus reduces its migration out of the absorbent article. Advantageously the shelf live of the absorbent articles is significantly prolonged thereby.

Without being bound by theory it is assumed that narrow silica gel can adsorb the most volatile and active compounds due to its porosity, particularly by creation of hydrogen bonding, and easily release active compounds during usage of product, i.e. due to presence of water that competes with the absorbed molecules in the formation of hydrogen bonds with the silica gel surface.

The stabilization effect has been proven by running a Thermogravimetric analysis (TGA) on samples of narrow silica gel treated with 10 weight-% of menthyl acetate. Specifically, the method is based on evaluation of weight loss over time at specific temperature (40°C). The samples were kept at 40 °C for 180 minutes. In order to have a basis for comparison the TGA analysis was performed on a sample of pure menthyl acetate and a sample of pure silica gel as references together with a sample of menthyl acetate (10 %) + silica gel (ex Grace, coded 123). The results are listed in table 1 and illustrate that the sample with silica gel and MA has a significant lower weight loss than the samples of menthyl acetate alone.

**Table 1**

| Data point | Sample composition | Weight Loss (%) |
|---|---|---|
| 1 | Menthyl acetate | ~100% |
| 2 | Silica gel + Menthyl acetate | 3 |
| 3 | Silica gel | 3 |

One component of body fluid malodour is ammonia. For example ammonia is present in high amounts in products used for urine absorption due to degradation of urea. Ammonia and its derivatives can react with aldehyde to form imines (according to the so-called Schiff base reaction).

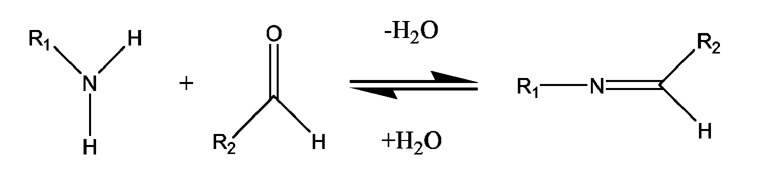

This reaction is catalyzed by enzymes and/or by a slightly acidic pH 4 to 5. The moderate acid requirement is necessary to allow protonation of the hydroxyl intermediate to allow water to leave.

Unfortunately, most aldehydes capable of imine reaction have an unpleasant and/or too intense odour that can be disturbing to human nose and/or they are very volatile and so not stable on the product.

Therefore, it is desirable to select suitable materials for controlling malodour. Examples of suitable aldehydes for controlling malodour are those aldehydes that are able to react with aminic compounds according to Schiff base reaction and have not unpleasant odour. Suitable aldheydes include α-amylcinnamic aldehyde, p-anisaldheyde, benzaldehyde, cinnamic aldehyde, cuminic aldehyde, decanal, p-t-Butyl-alpha-methyldihydrocinnamaldehyde, 4-Hydroxy-3-methoxycinnamaldehyde, 2-Phenyl-3-(2-furyl)prop-2-enal, Vanillin isobutyrate, 2-Phenyl-3-(2-furyl)prop-2-enal, Ethyl vanillin acetate, Vanillin acetate, cyclamen aldehyde, heptanal, lauryl aldehyde, nonanal, octanal, phenylacetaldehyde, phenyl propyl aldehyde, vanillin, salycil aldehyde, cytral and mixtures thereof Some of the most desirable aldehydes for application herein are alpha-amylcinnamaldehyde and decanal.

### Odour control materials acting on nose receptors

The second class of odour control materials in the odour control systems counteracts odours externally, outside the absorbent articles. Specifically, the materials listed hereinafter inhibit the receptors of the nose, hereinafter called "nose blocking". When used, these materials may significantly reduce the capability for the nose to detect the malodours.

The nose blocking is possible due to the volatile nature of the materials selected, which are evaporating out of the absorbent article and are then inhaled into the nose of an individual generally within somewhat close range of the article, e.g. within about 0 to 10 meters of the article (although this should in no way be intended to limit the scope of the invention) by normal breathing. The blocking of the nose receptors is of course only temporary.

Suitable noseblocking materials include menthol, menthyl acetate, 3-Buten-2-one,3-methyl-4-(2,6,6-trimethyl-2-cyclohexen-1-yl)-, 4-(2,6,6-Trimethylcyclohen-1-en-1-yl)but-3-en-2-one, 3-Buten-2-one,4-(2,6,6-trimethyl-2-cyclohexen-1-yl)-,(E)-, menthyl lactate, isomenthyl acetate, isomenthyl propionate, isomenthyl isobutyrate, isomenthyl propionate, isomenthyl butyrate, camphor and p-menthane. The materials also include their isomeric forms, diastereomers and enantiomers. Advantageously, in generally, the above materials have only a very slight inherent odour but show a high degree of nose receptor blocking.

### Optional further components

### a) High vapour pressure materials

The action of the odour control system of the present invention can be further improved by providing the absorbent article with a highly volatile component. According to the law of Dalton, due to their high partial pressure in the headspace of the absorbent article such highly volatile components are believed to reduce the molar fraction of the malodorous compounds having a lower partial pressure.

Suitable highly volatile components include materials that have a KI (Kovat Index) below 1500 and pleasant odour. The Kovat Index is defined by the selective retention of perfume raw materials (PRMs) onto chromatographic columns (30M X 0.25mm DB51u 50-300 @4 12.0 psi; Constant Flow, available from Agilent Technologies Inc (ex J&W Scientific)). A PRM's polarity, molecular weight, vapor pressure, boiling point, and the stationary phase property determine the extent of retention.

Suitable highly volatile components that act according to this mechanism include e.g. limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, Ethylhexanal, octanol acetate, methyl furfural, terpinene, thujene, amylacetate, benzylacetate, camphene, citronellal, dihydrocumarin, dy hydromyrcenyl acetate, geraniol, geranial, isoamylacetate, ethyl, and /or triethyl acetate, para-cresol and para-cymene.

### b) Solvents

Further additional ingredients include solvents as carriers for incorporating the odour control materials into the absorbent article. Suitable solvents are e.g. benzyl-benzoate, isopropyl myristate, methyl abietate, ethanol, isopropanol, diethylene glycol monoethyl ether, glycerol, propylene glycol, 1,2-butylene glycol, dipropylene glycol, 2-methyl-2,4-pentanediol, diethyl phthalate, trietyl citrate, diethyl sebacate.

### The odour control system

It has been found by the present inventors that the combination of certain, separately known odour control materials results in a synergistic effect in terms of odour reduction.

Specifically, a synergistic effect in terms of malodour reduction is observed when combining odour control materials acting on the malodours or malodorous substance itself and odour control materials acting on nose receptors, as described above. In fact, an odour control system combining actives of the aforementioned two classes may reduce the malodours more efficiently than the mathematical combination of each material acting individually.

Without wishing to be bound by theory this is believed to be a result of the neutralization of the malodorous molecules in the absorbent article by the odour control materials acting internally, which reduces the concentration of these molecules in the head space and thus, in the air. Due to this, the odour control material acting externally onto the nose receptors can exploit their maximum activity.

When the odour control system of the present invention is used in absorbent articles the individual odour control materials can be employed at variable amounts. Starting with the odour control material acting internally, for silica gel an amount of from 5 g/m² to 300 g/m², or from 20 g/m² to 100 g/m² has proven useful. For cinnamic aldehyde, an amount of from 0.05 g/m² to 20 g/m², or from 0.5 g/m² to 0.5 g/m² has proven useful. An example of an odour control material acting on nose receptors includes menthyl acetate in the range from 0.05 g/m² to 20 g/m², or from 0.5 g/m² to 5 g/m².

The odour control system comprises the first class of odour control material, acting on the malodours or malodorous substance, in relation to the second class of odour control material, acting on the nose receptors, at a ratio of from 50:1 to 1:50 by weight, or from 30:1 to 1:30 by weight or preferably from 1:15 to 15:1 by weight. In one embodiment, the odour control system comprises silica gel and menthyl acetate at a ratio of 15:1 by weight. In an alternative embodiment the odour control system comprises alpha-amylcinnamaldehyde and menthyl acetate at a ratio of 1:1 by weight.

### Absorbent article

The absorbent article being provided with the odour control system herein can be any kind of absorbent article of personal hygiene known in the art. The odour control system of the present invention can be present in any part of the absorbent article. According to the present invention the odour control system can be either be present in the absorbent article as an intimate mixture of the at least two classes of odour control materials or with both classes of odour control materials being separate from each other.

In one embodiment of the present invention the odour control system is present in the absorbent core. In another embodiment of the present invention the odour control system is present in or on the secondary topsheet, which might be present between the topsheet, which is the uppermost layer of the absorbent article, and the absorbent core. A further embodiment of the present invention has the first class of odour control materials (acting on the malodours or malodorous substance) placed on the wearer-facing surface of the absorbent core and the second class of odour control materials (acting on nose receptors) placed on the garment-facing surface of the absorbent core. Taking into account the stabilization described herein before, one execution of this embodiment is an absorbent article being provided with silica gel on the wearer-facing surface of the absorbent core and with menthyl acetate of the garment-facing surface of the absorbent core. However, other arrangements of the first and second odour control materials are contemplated including placing them in other elements of the article and/or providing one or more of them separate from the article.

### Test methods and data

For proving the synergistic effect on odour reduction several test samples have been prepared, which are all being exposed to a 0.1% aqueous solution of ammonia, which serves as a test malodorous substance. The first data point in table 2 serves as a benchmark as it represents the degree of unpleasantness of the ammonia solution without added odour control material. Data points 2-3 in table 2 are illustrating the malodour-reducing activity of two exemplary odour control materials alone, specifically alpha-amylcinnamaldehyde as exemplary aldehyde and menthyl acetate as exemplary odour control material acting on the nose receptors.

The last data point 4 in table 2 is illustrating the activity of the odour control system of the present invention. For comparability two individual odour control materials were mixed at half their amount compared to data points 2-3. Data point 4 has been obtained by testing a mixture of alpha-Amylcinnamaldehyde and menthyl acetate. It is clearly obtainable from data point 4 that the odour reduction performance of the odour control system is significantly better than the one of the individual odour control materials. Thus, synergistic odour control activity has been proven.

**Table 2**

| Data point # | Sample composition | Odor unpleasantness (%) |
|---|---|---|
| 1 | Malodorant solution (10 ml) | **100** |
| 2 | Alpha-Amylcinnamaldehyde (16 mg) + Malodorant solution (10 ml) | **64** |
| 3 | Menthyl acetate (16 mg) + Malodorant solution (10 ml) | **46** |
| 4 | Alpha-Amylcinnamaldehyde (8mg) + Menthyl acetate (8 mg) + Malodorant solution (10 ml) | **30** |

The odour unpleasantness and pleasantness of these samples have been evaluated by a panel of expert graders. In particular five different expert graders have evaluated 4 replicates for each sample. Odour unpleasantness have been evaluated by using a scale from -10 to + 0, where - 10 indicates the max odour unpleasantness, 0 indicates no odour. Data have been then reported as % relative unpleasantness vs. Reference (malodourant solution). The odour evaluation has been performed in adequate room, at controlled T (25°C). The room is equipped with appropriate conditioning system allowing continuous exchange of air. The samples were held in numbered metal trays, which were covered with aluminium foil between the actual gradings.

All documents cited in the Detailed Description of the Invention are, in relevant part, incorporated herein by reference; the citation of any document is not to be construed as an admission that it is prior art with respect to the present invention. To the extent that any meaning or definition of a term in this written document conflicts with any meaning or definition of the term in a document incorporated by reference, the meaning or definition assigned to the term in this written document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

Each dimension for which a value is defined herein is a technical dimension, which, in the context of the present invention is not to be understood literal. Hence, all embodiments having dimensions functionally equivalent to the dimensions stated herein are intended to be covered by the scope of the invention, e.g. a dimension of "40 mm" has to be understood as meaning "about 40 mm".

## Claims

1. An absorbent article comprising an odour control system, wherein the odour control composition comprises two classes of odour control material, wherein a first class of odour control material reduces odour by acting on malodours or a malodorous substance in the absorbent article and a second class of odour control material reduces odour by acting on the user's nose receptors, the absorbent article **characterized in that** the first class of odour control material is selected from the group consisting of silica gel having a pH of less than 7, aldehydes, mesoporous zeolites having pores sizes of from 20 to 500 Å and mixtures thereof, wherein said aldehydes are selected from the group consisting of α-amylcinnamic aldehyde, p-anisaldheyde, benzaldehyde, cinnamic aldehyde, cuminic aldehyde, decanal, cyclamen aldehyde, p-t-Butyl-alpha-methyldihydrocinnamaldehyde, 4-Hydroxy-3-methoxycinnamaldehyde, 2-Phenyl-3-(2-furyl)prop-2-enal, Vanillin isobutyrate, 2-Phenyl-3-(2-furyl)prop-2-enal, Ethyl vanillin acetate, Vanillin acetate, heptanal, lauryl aldehyde, nonanal, octanal, phenylacetaldehyde, phenyl propyl aldehyde, vanillin, salycil aldehyde, cytral and mixtures thereof, and the second class of odour control material is selected from the group consisting of menthol, menthyl acetate, menthyl lactate, 3-Buten-2-one,3-methyl-4-(2,6,6-trimethyl-2-cyclohexen-1-yl)-, 4-(2,6,6-Trimethylcyclohen-1-en-1-yl)but-3-en-2-one, 3-Buten-2-one,4-(2,6,6-trimethyl-2-cyclohexen-1-yl)-,(E)-, isomenthyl acetate, isomenthyl propionate, isomenthyl isobutyrate, isomenthyl propionate, isomenthyl butyrate, camphor, p-menthane and mixtures thereof.

2. The absorbent article of claim 1, wherein the silica gel has a pH of less than 6.

3. The absorbent article of claim 1, wherein the silica gel has a pore size of from 20 to 40 Å, preferably 30Å.

4. The absorbent article of any of the previous claims, wherein the silica gel has a total surface area higher than 500 m²/g.

5. The absorbent article of any of the previous claims, wherein the odour control system further comprises a highly volatile component selected from the group consisting of limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, Ethylhexanal, octanol acetate, methyl furfural, terpinene, thujene, amylacetate, benzylacetate, camphene, citronellal, dihydrocumarin, dy hydromyrcenyl acetate, geraniol, geranial, isoamylacetate, ethyl, and /or triethyl acetate, para-cresol, para-cymene, methyl abietate and mixtures thereof

6. The absorbent article of any of the previous claims, wherein the odour control system comprises narrow silica gel in an amount of from 5 g/m² to 300 g/m², preferably from 20 g/m² to 100 g/m².

7. The absorbent article of any of the previous claims, wherein the odour control system comprises alpha-amylcinnamaldehyde in an amount of from 0.05 g/m² to 20 g/m², preferably from 0.5 g/m² to 5 g/m².

8. The absorbent article of any of the previous claims, wherein the odour control system comprises menthyl acetate in an amount of from 0.05 g/m² to 20 g/m², preferably from 0.5 g/m² to 5 g/m².

9. The absorbent article of any of the previous claims, wherein the odour control system comprises the first class of odour control material in relation to said second class of odour control material at a ratio of from 50:1 to 1:50 by weight, preferably from 30:1 to 1:30 by weight, and more preferably from 1:15 to 15:1 by weight.

10. The absorbent article of claim 9, wherein the odour control system comprises narrow silica gel and menthyl acetate at a ratio of 15:1 by weight.

11. The absorbent article of claim 9, wherein the odour control system comprises alpha-amylcinnamaldehyde and menthyl acetate at a ratio of 1:1 by weight.
